# EUROPEAN PATENT SPECIFICATION

(11) **EP 3 438 644 B1**
(45) Date of publication and mention of the grant of the patent: **23.12.2020**
(21) Application number: 17774314.3
(22) Date of filing: 15.03.2017
(51) Int. Cl.: G01N 15/14, C12Q 1/02, G01N 21/27, G01N 21/64, G01N 33/48, G01N 33/483, G06K 9/00, H04N 5/235, G01N 15/10

(54) **CELL ANALYSIS SYSTEM**
ZELLANALYSESYSTEM
SYSTÈME D'ANALYSE CELLULAIRE

(30) Priority: 28.03.2016 JP 2016064094
(43) Date of publication of application: 06.02.2019
(73) Proprietor: FUJIFILM Corporation, Tokyo 106-8620 (JP)
(72) Inventor: MATSUBARA, Kenta, Kanagawa 258-8538 (JP)
(74) Representative: Klunker IP Patentanwälte PartG mbB
(86) International application number: PCT/JP2017/010359
(87) International publication number: WO 2017/169770

(56) References cited:
- EP-A1- 2 637 015
- WO-A1-2015/056516
- JP-A- 2001 526 051
- JP-A- 2005 257 450
- JP-A- 2010 510 782
- JP-A- 2010 535 013
- JP-A- 2015 078 927
- JP-A- 2015 152 439
- US-A1- 2002 102 570
- US-A1- 2002 149 763
- US-A1- 2011 081 684
- US-A1- 2016 041 144

## Description

### BACKGROUND OF THE INVENTION

### 1. Field of the Invention

The present invention relates to a cell analysis system.

### 2. Description of the Related Art

It is inefficient to analyze all cells (millions of cells) in order to analyze the exhaustive gene expression of a specimen with high accuracy. Therefore, the expression state of a specific gene or protein of a cell is measured and sorted. For example, a cell analysis apparatus using flow cytometry which stains a cell with a fluorochrome bound to the gene or protein of the cell, irradiates a sample solution including the cell with laser light, and measures intensity to sort a target cell has been known.

JP2003-530899A discloses a technique which investigates the simultaneous expression of a maximum of four types of cell surface markers on an individual donor cell, using flow cytometry, and sorts a target cell group with a cell sorting device in order to confirm that the cell results from the donor.
US 2002/0149763 A1 discloses an analytical device comprising means for centrifuging a plate-like sample container; means for generating a laser beam; means for scanning the sample container and irradiating a plurality of particulate substances having been centrifuged in the sample container with a laser beam; and means for detecting scattered light from the sample container.

### SUMMARY OF THE INVENTION

In general, in a case in which a target cell is sorted by flow cytometry, cells are dropped into each well of a container that is called a multi-well plate and are collected. The polymerase chain reaction (PCR) occurs in the multi-well plate into which the cells have been collected and gene analysis is performed.

However, in the flow cytometry, in some cases, since the cells are sorted on the basis of the obtained optical information, dust or a cell other than the target cell is collected into the well. It is considered that each well of the multi-well plate is observed by, for example, a microscope in order to remove dust or the cell other than the target cell. However, since it takes a lot of time to observe the multi-well plate, it is difficult to keep the cell in the well fresh.

In a case in which a cell is not fresh, it is difficult to perform gene analysis, which causes an error in gene analysis.

In addition, since gene analysis costs a lot, it is preferable to perform gene analysis for the target cell that is kept fresh.

The invention has been made in view of the above-mentioned problems and an object of the invention is to provide a cell analysis system that can keep only a target cell fresh.

According to an aspect of the invention, a cell analysis system comprises: a detection device that detects staining intensity of a stained cell; a collection container that collects a plurality of cells whose staining intensity has been detected by the detection device; a control unit that is configured to determine priority in acquiring image data on the basis of a detection result of the staining intensity of the stained cell; and an imaging apparatus that acquires the image data of the cells collected into the collection container on the basis of the priority determined by the control unit.

Preferably, the control unit determines image data acquisition conditions on the basis of the detection result of the staining intensity of the stained cell.

Preferably, the control unit limits the number of cells whose image data is to be acquired, on the basis of the detection result of the staining intensity of the stained cell and the number of cells collected into the collection container.

Preferably, the control unit limits the number of cells whose image data is to be acquired, on the basis of the detection result of the staining intensity of the stained cell and a time for which the cells are collected into the collection container.

Preferably, the cell is stained with a plurality of types of dyes.

Preferably, the control unit determines whether to analyze the cells collected into the collection container on the basis of the image data.

Preferably, the cell is a blood cell.

Preferably, the conditions include at least one of an exposure time in a case in which the image data is acquired, an observation magnification, a wavelength of illumination light, or a wavelength of observation light.

Preferably, at least one of the shape, color, size, or staining intensity of the cell is analyzed on the basis of the image data.

According to the cell analysis system of the invention, it is possible to keep only a target cell fresh and to effectively perform cell analysis.

### BRIEF DESCRIPTION OF THE DRAWINGS

Fig. 1 is a diagram schematically illustrating the configuration of a cell analysis system.
Fig. 2 is a diagram illustrating the principle of a detection device.
Fig. 3A is an enlarged perspective view illustrating a collection container.
Fig. 3B is an enlarged perspective view illustrating the collection container.
Fig. 4 is a diagram schematically illustrating the configuration of an imaging apparatus.
Fig. 5 is a scattergram in which the vertical axis indicates BSC-A and the horizontal axis indicates FSC-A.
Fig. 6 is a scattergram in which the vertical axis indicates FSC-A and the horizontal axis indicates Brilliant Violet 421 of a CD45 antibody.
Fig. 7 is a scattergram in which the vertical axis indicates FITC of a CD71 antibody and the horizontal axis indicates APC of DRAQ5.
Fig. 8 is a plan view illustrating the collection container placed on the imaging apparatus.
Fig. 9 is an image diagram in which the fluorescent color of a dye labeling a cell collected in a well is binarized.
Fig. 10A illustrates image data of a specific well in the collection container.
Fig. 10B illustrates image data of another well in the collection container.

### DESCRIPTION OF THE PREFERRED EMBODIMENTS

Hereinafter, a preferred embodiment of the invention will be described with reference to the accompanying drawings. The invention is described by the following preferred embodiment. The invention can be changed by many methods without departing from the scope of the invention and embodiments other than this embodiment can be used. Therefore, all changes in the scope of the invention are included in the scope of the claims.

Here, in the drawings, portions denoted by the same reference numerals are the same components having the same functions. In addition, in the specification, in a case in which a numerical range is represented by "to", the numerical range includes the upper limit and the lower limit represented by "to".

### <Cell Analysis System>

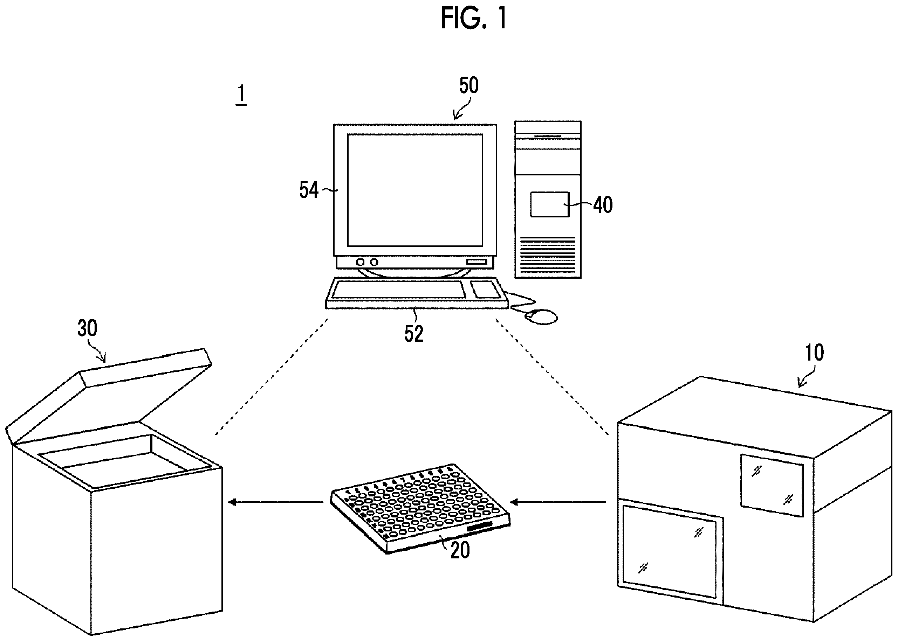

An embodiment of the invention will be described with reference to Figs. 1 to 4. Fig. 1 is a diagram schematically illustrating the configuration of a cell analysis system.

A cell analysis system 1 includes a detection device 10, a collection container 20, an imaging apparatus 30, and a personal computer 50 including a control unit 40. The personal computer 50 is electrically connected to the detection device 10 and the imaging apparatus 30. The personal computer 50 including the control unit 40 controls the overall operation of the cell analysis system 1. The personal computer 50 includes a keyboard 52 which is an operation input unit and a display 54 which is a display unit. In addition, the control unit 40 includes, for example, an arithmetic unit that performs various processes and a storage unit that stores various programs and data.

The detection device 10 is a device that can detect the staining intensity of a stained cell. A flow cytometer can be given as an example of the detection device 10. The flow cytometer means a device that is used for flow cytometry. In addition, the stained cell means a cell to which a chemical having stain information, such as a pigment or a dye, has been bound. A preferred aspect of the stained cell is a cell to which a fluorochrome-labeled antibody has been bound. The staining intensity is information related to the structure or function of the cell. A preferred aspect of the staining intensity is the intensity of fluorescent light from a fluorochrome that is specifically bound to a nucleus. The staining intensity is information related to the nucleus.

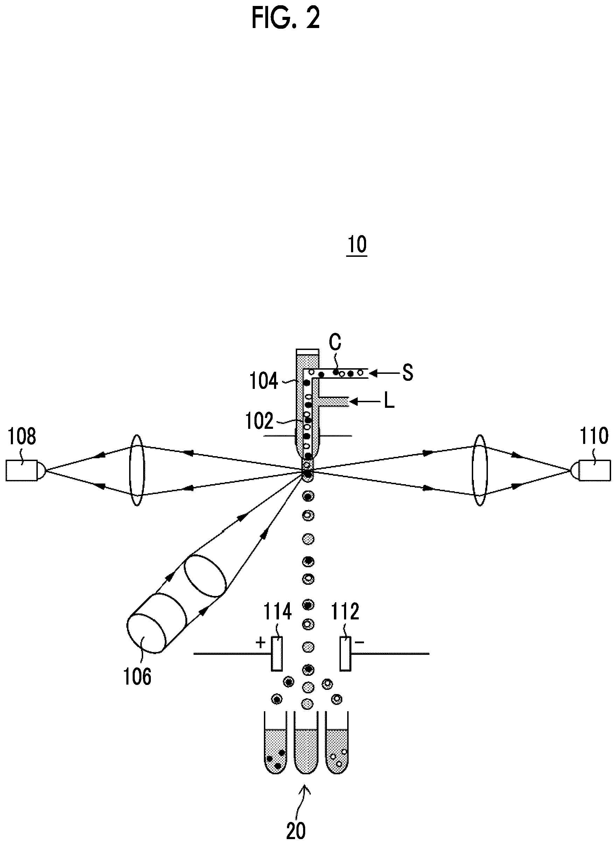

The principle of the detection device 10 will be described in brief with reference to Fig. 2. An analysis target sample includes a cell C to which a fluorochrome-labeled antibody has been bound. A sample solution S including the cell C is introduced from a nozzle 102 to a flow cell 104. In addition, a sheath solution L is introduced into the flow cell 104. In the flow cell 104, the sample solution S is narrowed such that the cells C are arranged in a line. The cell C is irradiated with, for example, laser light from a light source 106. The fluorochrome labeling the cell C is irradiated with the laser light, is excited, and emits fluorescent light. The detector 108 detects the intensity of the fluorescent light. The detection result of the fluorescence intensity of the cell C is input to the control unit 40 and is stored in the control unit 40.

In addition, a detector 110 detects scattered light (for example, forward scattered light and side scattered light) from the cell C irradiated with the laser light. The detection result of the fluorescence intensity by the scattered light from the cell C is input to the control unit 40 and is stored in the control unit 40. The size of a measurement target cell can be measured by the forward scattered light and the structure of the measurement target cell can be measured by the side scattered light and the fluorescent light.

A liquid droplet including the cell C to which ultrasonic waves are applied is formed in the flow cell 104. The control unit 40 charges the liquid droplet with positive or negative electricity on the basis of the detection results. The control unit 40 does not charge the liquid droplet to be discarded with electricity. In a case in which the liquid droplet passes between deflection electrode plates 112 and 114, the liquid droplet is drawn to any one of the deflection electrode plates 112 and 114. In this way, the charged liquid droplets are sorted and are collected into the collection container 20.

A plurality of laser light sources with different wavelengths can be used as the light source 106 that excites the fluorochrome. For example, it is preferable to include a laser light source with a wavelength of 405 nm, a laser light source with a wavelength of 488 nm, a laser light source with a wavelength of 561 nm, and a laser light source with a wavelength of 683 nm.

It is preferable that a fluorescence filter which cuts the excitation light of the laser light source and selectively transmits a fluorescence wavelength from the fluorochrome is used in order to detect the fluorescence intensity at the same.

In addition, the cell C can be stained with a plurality of fluorochromes. For example, in a case in which the cell is a human blood cell, it is preferable that antibodies which are specifically bound to antigens of the blood cell, such as CD3, CD4, CD14, CD25, and CD127, are labeled with fluorochromes and the labeled antibodies are bound to the cell to stain the cell. In addition, it is preferable to stain the cell with 4',6-diamidino-2-phenylindole (DAPI) which is a fluorochrome bound to a deoxyribonucleic acid (DNA) of a nucleus. It is preferable to determine a combination of the excitation wavelength of the light source 106 used and the fluorescence filter, depending on the fluorochrome labeling the antibody specifically bound to the antigen of the cell C or the fluorochrome staining the DNA.

Examples of other fluorochromes that can be used in this embodiment include propidium iodide (PI), pyronin Y, fluorescein isothiocyanate (FITC), phycoerythrin (PE), allophicocyanin (APC), Texas Red (TR (registered trademark)), Hoechst33342, 7-aminoactinomycin D (7-AAD), 2'-deoxycytidine5'-triphosphate (Cy3), sulfoindocyanine succinimidyl ester (Cy5), DRAQ5 (registered trademark), Brilliant Violet 570, Brilliant Violet 421, and a plurality of stain types.

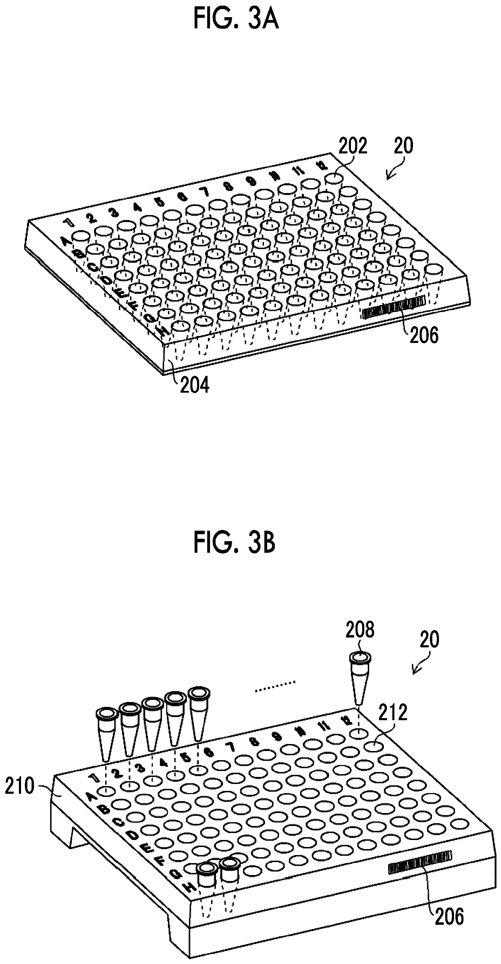

Figs. 3A and 3B are enlarged perspective views illustrating the collection container 20. As illustrated in Fig. 3A, the collection container 20 includes a plurality of wells 202, each of which has an opening and a bottom, and a side wall 204 which is formed integrally with the wells 202 in order to collect a plurality of cells. The plurality of wells 202 are arranged in the row and column directions. In order to specify the position of each well 202, numbers indicating rows and English letters indicating columns are displayed on the opening side of the wells 202 in the collection container 20. In the collection container 20 illustrated in Fig. 3A, cells are collected into each well 202. In addition, in order to specify the collection container 20, for example, an identification mark 206, such as a bar code, is displayed on the side wall of the collection container 20.

As illustrated in Fig. 3B, the collection container 20 having a shape different from that illustrated in Fig. 3A includes a plurality of tubes 208 which are used to collect a plurality of cells and each of which has an opening and a bottom and a support member 210 which has a plurality of holes 212 for supporting the plurality of tubes 208.

The plurality of holes 212 are formed in the row and column directions. In order to specify the position of each hole 212, value indicating rows and English letters indicating columns are displayed on the side on which the holes 212 are formed in the collection container 20. In the collection container 20 illustrated in Fig. 3B, cells are collected into each tube 208 held by the support member 210. In addition, in order to specify the collection container 20, for example, the identification mark 206, such as a bar code, is displayed on the side wall of the support member 210. The tube 208 may be, for example, a single tube or a plurality of tubes may be continuous with each other. In addition, the tube 208 may have a cap (not illustrated).

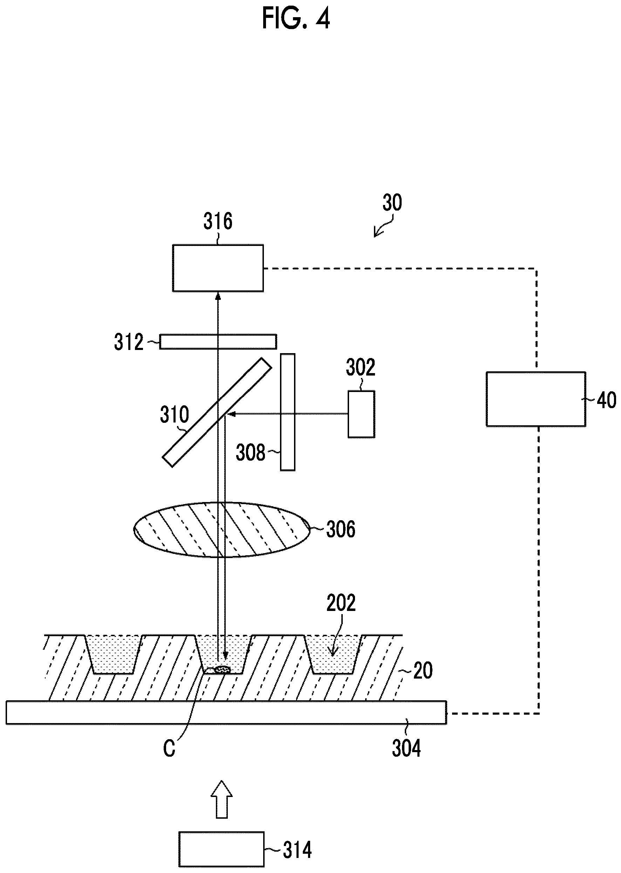

A microscope including an imaging device can be given as an example of the imaging apparatus 30. Fig. 4 is a diagram schematically illustrating the configuration of the imaging apparatus 30. The imaging apparatus 30 can acquire the image data of the cell C collected into the collection container 20. The image data of the cell C includes at least one of image data obtained by capturing fluorescent light emitted from the stained cell C or image data of a bright field obtained by capturing a light transmission image of the cell C.

The imaging apparatus 30 includes a first light source 302 for excitation that measures fluorescent light from the cell C, a table 304 on which the collection container 20 is placed, a lens 306 that is provided on the side of the wells 202 of the collection container 20, a filter group including an excitation filter 308, a dichroic mirror 310, and a fluorescence filter 312, a second light source 314 that is provided on the side opposite to the wells 202 of the collection container 20, and an imaging device 316 that captures fluorescent light and transmitted light from the cell C. The second light source 314 irradiates the cell C with light for measuring transmitted light.

For example, a high-pressure mercury lamp, a high-pressure xenon lamp, a light emitting diode, a laser diode, a tungsten lamp, a halogen lamp, and a white light emitting diode can be used as the first light source 302. Even in a case in which these light sources are used, the excitation filter 308 can transmit only a target wavelength. It is possible to irradiate the fluorochrome labeling the antibody bound to the cell C with light having a target excitation wavelength. In addition, the same light source as the first light source 302 can be used as the second light source 314.

A case in which the imaging device 316 acquires image data in order to obtain the intensity of fluorescent light from the fluorochrome labeling the antibody bound to the cell C will be described. The excitation filter 308 transmits only light with a target wavelength range among light components emitted from the first light source 302. The light transmitted through the excitation filter 308 is reflected to the collection container 20 by the dichroic mirror 310. The light reflected from the dichroic mirror 310 is transmitted through the lens 306 and the cell C collected in the well 202 is irradiated with the light. The light emitted to the cell C has a wavelength range that excites the fluorochrome labeling the antibody bound to the cell C. The fluorochrome labeling the antibody bound to the cell C is excited by the light and emits fluorescent light with a wavelength different from the excitation wavelength of the emitted light. The fluorescent light from the fluorochrome labeling the antibody bound to the cell C is transmitted through the lens 306, the dichroic mirror 310, and fluorescence filter 312 and is captured by the imaging device 316 and image data is acquired. The acquired fluorescence image data is input to the control unit 40 and is stored in the control unit 40. In a case in which the excitation light is compared with the fluorescent light emitted by the excitation light, the fluorescent light has a longer wavelength than the excitation light. Therefore, the dichroic mirror 310 can reflect light having the wavelength of the excitation light to the collection container 20 and can transmit light having the wavelength of the fluorescent light to the imaging device 316. In addition, the fluorescence filter 312 does not transmit the excitation light and can transmit only the fluorescent light. Therefore, the imaging device 316 can capture the fluorescent light from the fluorochrome labeling the antibody bound to the cell C. Since the fluorescence filter 312 transmits only the fluorescent light, the image captured by the imaging device 316 is not affected by the excitation light. Therefore, it is possible to acquire accurate image data.

The imaging apparatus 30 according to this embodiment includes the table 304 and a driving device (not illustrated) in order to move the collection container 20 to any position (for example, the X direction, the Y direction, and the Z direction). The table 304 and the driving device can move a specific well 202 of the collection container 20 to an observation position. Preferably, the driving device can move the table 304 in the X direction, the Y direction, and the Z direction.

In a case in which a plurality of antibodies labeled with different fluorochromes are bound to the cell C, the filter group can be switched to a different filter group (the excitation filter 308, the dichroic mirror 310, and the fluorescence filter 312) to acquire the image data of light with a different fluorescence wavelength.

In a case in which light that has been emitted from the second light source 314 and then transmitted through the cell C is captured, the light is captured in a state in which the filter group is removed. The imaging device 316 captures the transmitted light to acquire the image data of a bright field. The acquired image data of the bright field is input to the control unit 40 and is stored in the control unit 40.

### <Operation of Cell Analysis System>

Next, the operation of the cell analysis system 1 will be described. Peripheral blood including blood cells, two types of fluorochrome-labeled antibodies (for example, a CD45 antibody labeled with Brilliant Violet 421 and a CD71 antibody labeled with FITC) for staining cells, and DRAQ5 and APC which are anthraquinone dyes with a high affinity for a double-stranded DNA are prepared as analysis target samples, are mixed with each other, and are incubated to stain the blood cells.

The analysis target samples are set in the detection device 10 and the intensity of fluorescent light (fluorescent light, forward scattered light, and side scattered light) from the fluorochrome of the cell, to which the fluorochrome-labeled antibody has been bound, is detected by flow cytometry. The fluorescence intensity detected for each cell is input to the control unit 40. The control unit 40 stores the cells and the detected fluorescence intensity so as to be associated with each other. An analysis program is stored in the control unit 40. The analysis program can be used to perform analysis on the basis of each cell and fluorescence intensity data.

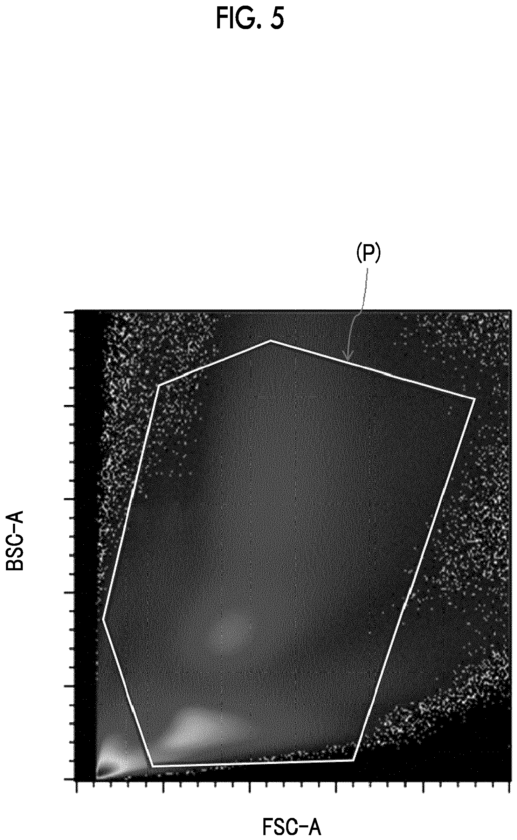

For example, as illustrated in Fig. 5, the control unit 40 creates a scattergram, in which the vertical axis indicates BSC-A which is the relative value of the intensity of side scattered light and the horizontal axis indicates FSC-A which is the relative value of the intensity of forward scattered light, from the fluorescence intensity of each cell. The control unit 40 gets (acquires) a nucleated red blood cell from the scattergram and demarcates a region (P) in which the nucleated red blood cell appears.

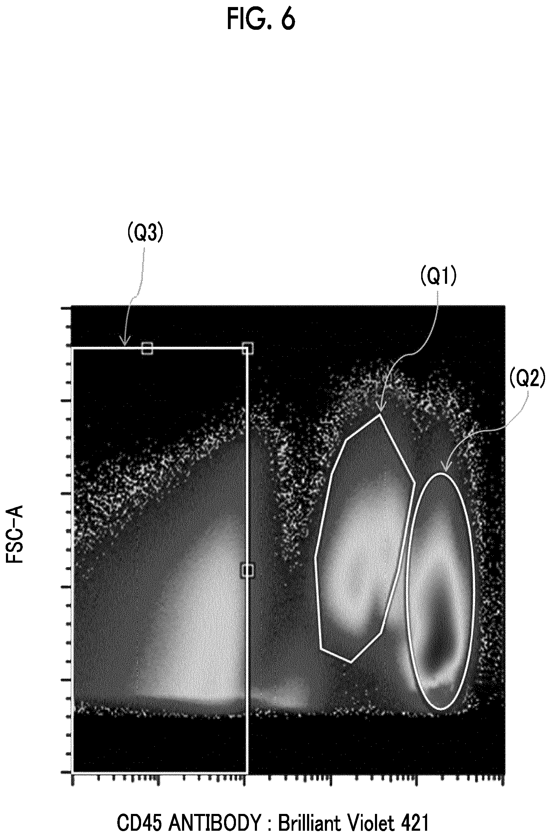

As illustrated in Fig. 6, the control unit 40 creates a scattergram, in which the vertical axis indicates FSC-A and the horizontal axis indicates CD45 antibody: Brilliant Violet 421, for the acquired nucleated red blood cell. The control unit 40 gets out (extracts) regions (Q1) and (Q2) in which white blood cells appear from the acquired nucleated red blood cell. The control unit 40 demarcates a region (Q3) in which the nucleated red blood cell appears except the white blood cell.

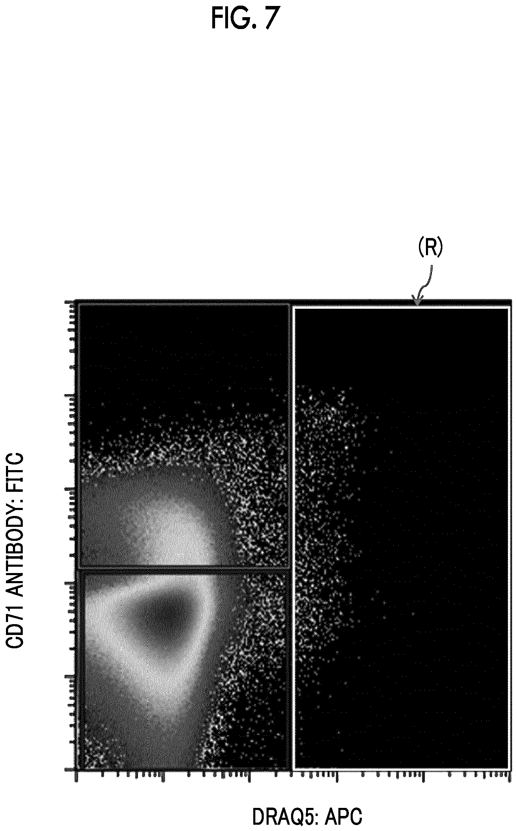

As illustrated in Fig. 7, the control unit 40 creates a scattergram, in which the vertical axis indicates the relative value of the fluorescence intensity of FITC of the CD71 antibody and the horizontal axis indicates the relative value of the fluorescence intensity of DRAQ5 (anthraquinone dye) that is the information of a nucleus, for the nucleated red blood cell from which the white blood cell has been extracted. Since the wavelength range overlaps the wavelength ranges of other fluorochromes, the amount of leakage to the detectors with each wavelength is compensated. The control unit 40 demarcates a region (R) in which the nucleated red blood cell that is positive for CD71 and DRAQ5 appears from the acquired nucleated red blood cell. Finally, the nucleated red blood cell that is positive for CD71 is measured.

The detection device 10 analyzes the cell, to which the fluorochrome-labeled antibody has been bound, on the basis of the fluorescence intensity. In the cell to which the fluorochrome-labeled antibody has been bound, a region (R) in which the nucleated red blood cell that is positive for CD71 is demarcated by the scattergrams illustrated in Figs. 5 to 7 on the basis of the analysis result.

In the description of the scattergram, positivity means that fluorescence intensity is high and negativity means that fluorescence intensity is low.

For example, about 500 cells included in the region (R) are collected into a plurality of collection containers 20 each of which has 96 wells 202. One cell is collected into one well.

In a case in which about 500 cells are collected into the plurality of collection containers 20, identification marks indicating the collection containers 20, the positions of the wells 202 of the collection containers 20, and the fluorescence intensity of each cell are associated with each other and data related to the association is input and stored in the control unit 40.

It is possible to perform gene analysis for all of the cells collected into the plurality of collection containers 20, without observing the images of the cells. However, in this case, costs increase. In some cases, cells other than a target cell are collected.

It is possible to observe the images of all of the cells collected into the plurality of collection containers 20. However, in a case in which the images of all of the cells are observed, it takes a lot of time and the freshness of the cells is likely to be reduced. As a result, in some cases, it is difficult to maintain the accuracy of gene analysis at a high level.

For this reason, in this embodiment, in the region (R) in which the cells are collected, a region in which the target cell is likely to be present is estimated on the basis of the detection result of the staining intensity of the cells. The control unit 40 determines priority in acquiring image data for the region in which the target cell is likely to be present.

As a method for determining the priority, about 500 cells are ranked in descending order of the fluorescence intensity of CD71 antibody: FITC and the fluorescence intensity of DRAQ5 in the region (R). Then, the control unit 40 determines the priority in acquiring image data on the basis of the ranking information.

In this embodiment, the case in which the cells are ranked in descending order of the fluorescence intensity of FITC of the CD71 antibody and the fluorescence intensity of DRAQ5 has been described. However, the invention is not limited thereto. For example, the cells may be ranked on the basis of α×the fluorescence intensity of FITC of the CD71 antibody+β×the fluorescence intensity of DRAQ5 (α and β are predetermined coefficients) and a weight for the fluorescence intensity may be changed. The coefficients α and β can be determined from the total number of cells collected by flow cytometry and the number of cells which have not been collected. The priority ranking can be changed as follows: in a case in which importance is attached to the weakness of the red blood cell, the coefficient α of the fluorescence intensity of FITC of the CD71 antibody increases; and, in a case in which importance is attached to the detection of a nucleus, the fluorescence intensity of DRAQ5 increases.

The plurality of collection containers 20 are transported from the detection device 10 to the imaging apparatus 30. Means for transporting the collection containers 20 to the imaging apparatus 30 is not particularly limited.

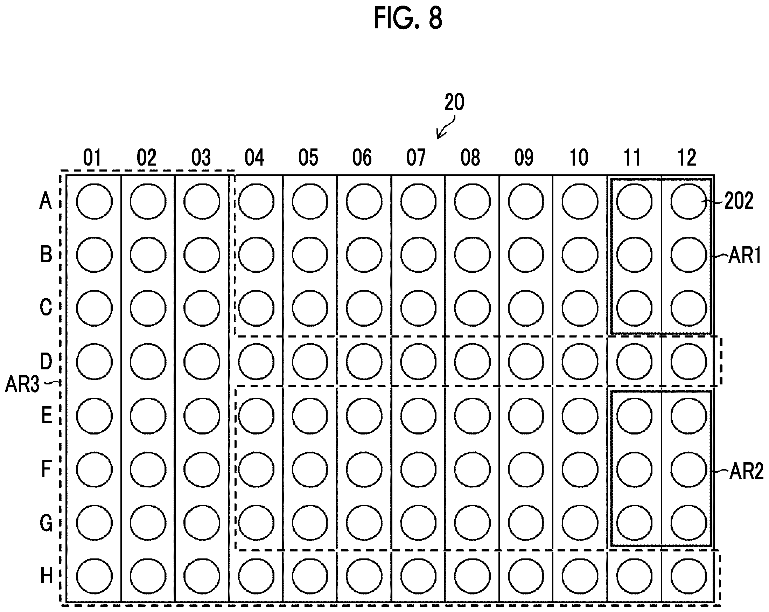

The collection container 20 is placed on the table 304 of the imaging apparatus 30. Fig. 8 is a plan view illustrating the collection container 20 placed on the table 304 of the imaging apparatus 30. In a case in which the collection container 20 is placed on the table 304, the identification mark 206 of the collection container 20 is read by, for example, a bar code reader and the information of the collection container 20 is input to the control unit 40. The information of the collection container 20 may be input from the keyboard 52 to the control unit 40.

In a case in which the collection container 20 is placed on the table 304, information, such as information related to the shape of the collection container 20, the outer size of the collection container 20, the diameter of the opening of the well 202, the pitch between the wells 202, and the number of wells 202, is input to the control unit 40.

The control unit 40 reads the fluorescence intensity of the cell collected into the well 202 and the ranking information from the information of the collection container 20. The control unit 40 specifies the position of the well 202 with high priority in the collection container 20 on the basis of the read information. In this embodiment, the control unit 40 specifies that the wells 202 with high priority are present in a region AR1 (All, A12, B11, B12, C11, and C12) surrounded by a solid line and a region AR2 (E11, E12, F11, F12, G11, and G12) surrounded by a solid line. In contrast, the control unit 40 specifies that the wells 202 with low priority are present in a region AR3 (01, 02, 03, D04 to D12, and H04 to H12) surrounded by a dashed line. The control unit 40 drives the table 304 to move an imaging target well 202 to the observation position on the basis of the priority. In this embodiment, the control unit 40 moves the well 202 located at A11 to the observation position and acquires the image data of the cell from the imaging device 316 of the imaging apparatus 30. The acquired image data is input to the control unit 40. The control unit 40 stores the image data.

The control unit 40 determines whether the cell whose image data is to be acquired is present in the collection container 20. In a case in which the control unit 40 determines that the cell whose image data is to be acquired is present, the control unit 40 drives the table 304 to move, for example, the well 202 which is located at A12 and whose image data has not been acquired to the observation position. The imaging device 316 of the imaging apparatus 30 acquires the image data of the cell. The acquired image data is input to the control unit 40. The control unit 40 stores the image data.

The control unit 40 repeats the movement of the well 202 and the acquisition of the image data until the control unit 40 determines that there is no cell whose image data is to be acquired.

In this embodiment, the imaging apparatus 30 acquires the image data of only six wells 202 among 96 wells 202 of the collection container 20 and ends the acquisition of the image data of the placed collection container 20.

As described above, in this embodiment, it is not necessary to acquire the image data of all of the cells collected into the wells 202 of the collection container 20. Therefore, it is possible to reduce the time required to acquire image data.

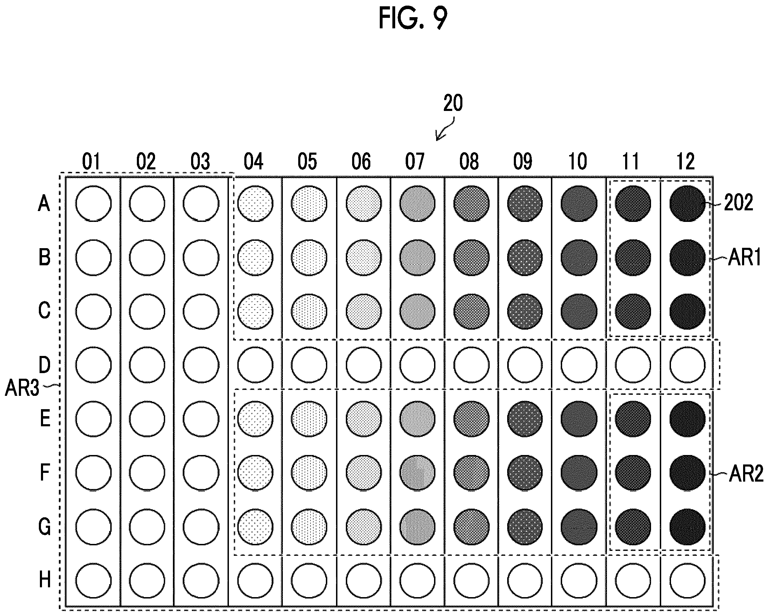

Fig. 9 is an image diagram in which the fluorescent color of a dye labeling the cell collected in the well 202 is binarized. In the image diagram, the fluorescent color of the cell in the well 202 is reproduced on the basis of the staining intensity (fluorescence intensity) of the cell acquired by the detection device 10.

In Fig. 9, it is estimated that the possibility of the target cell being collected into the well 202 with a dark color is high and the possibility of the target cell being collected into the well 202 with a light color is low.

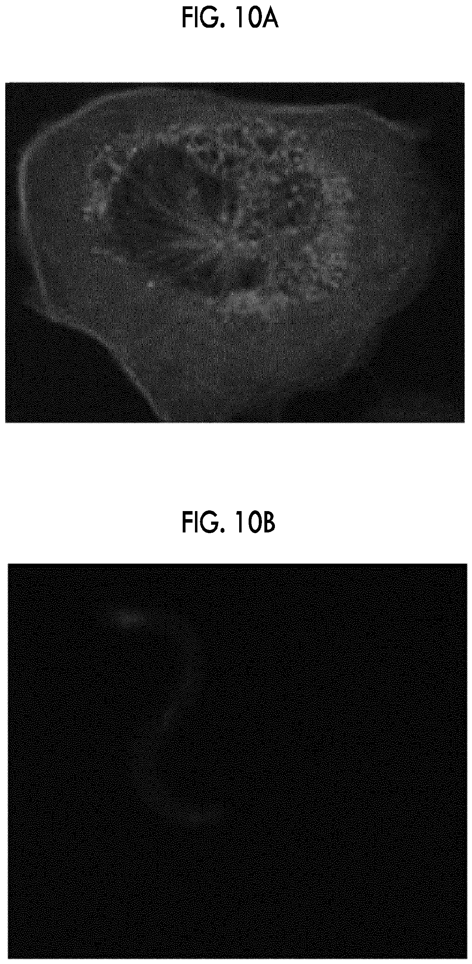

Fig. 10A illustrates the image data of the well 202 located at G12 and Fig. 10B illustrates the image data of the well 202 located at G02. As illustrated in Fig. 10A, the emission of fluorescent light components of a plurality of colors and the outline of the cell are confirmed by the captured image data of the well 202 located at G12. It can be determined that the possibility of the target cell being collected in the well 202 located at G12 is high. As illustrated in Fig. 10B, the emission of fluorescent light and the outline of the cell are not confirmed by the captured image data of the well 202 located at G02. It can be determined that dust or a dead cell is collected in the well 202 located at G02. In this embodiment, it is possible to acquire the image data of the target cell with high probability.

The imaging apparatus 30 acquires the image data of the fluorescence intensity of the cell. It is preferable that the control unit 40 determines image data acquisition conditions on the basis of the detection result. It is preferable that the control unit 40 determines at least one of exposure time in a case in which image data is acquired, an observation magnification, the wavelength of illumination light, or the wavelength observation light. An example of the determination will be described.

For example, in a case in which a cell is stained with Cy5 (sulfoindocyanine succinimidyl ester) for multi-immunostaining, the detection device 10 irradiates the cell with light in a wavelength range of 650 nm as excitation light. Fluorescent light in a wavelength range of 670 nm is emitted from the cell which has been excited by the light with a wavelength of 650 nm and then stained with Cy5. The detection device 10 detects the intensity of the fluorescent light in a wavelength range of 670 nm using a detector (for example, a photomultiplier tube (PMT)). The intensity of the fluorescent light emitted from the cell is estimated from the detection result.

For example, the control unit 40 sets the exposure time to 100 msec and sets the magnification to 20. In a case in which the exposure time increases, brightness increases. In a case in which the magnification increases, brightness increases. In addition, the control unit 40 selects an excitation filter that transmits light in a wavelength range of 650 nm which becomes the excitation light. Here, the excitation light is illumination light that illuminates the cell and the wavelength of the illumination light is determined. The control unit 40 selects a fluorescence filter that does not transmit the excitation light and transmits light in a wavelength range of 670 nm which is the wavelength range of fluorescent light. Here, the fluorescent light is emitted from the cell and becomes observation light observed by the imaging device 316. Therefore, the wavelength of the observation light is determined. As described above, since the image data acquisition conditions are determined on the basis of the detection result of the fluorescence intensity, it is possible to acquire image data with high accuracy. The illumination light and the observation light may have different wavelengths or the same wavelength.

It is preferable that the control unit 40 limits the number of cells whose image data is to be acquired on the basis of the detection result of the staining intensity of the stained cell and the number of cells collected into the collection container 20. For example, in some cases, the number of cells to be subjected to gene analysis has been determined in advance. In a case in which the number of cells desired to be subjected to gene analysis is reached, it is preferable that the acquisition of the image data ends, without acquiring the image data of all of a plurality of cells with high priority. According to this embodiment, the image data of only a predetermined number of cells among a plurality of cells with high priority may be acquired and the image data of all of the cells may not be acquired. The number of cells whose image data is to be acquired is limited to reduce the time required to acquire the image data.

In addition, it is preferable that the control unit 40 limits the number of cells whose image data is to be acquired on the basis of the detection result of the staining intensity of the stained cell and the time for which the cells are collected into the collection container 20. In some cases, the freshness of the cell is required to perform gene analysis. Even in a case in which a plurality of cells with high priority are collected into the collection container 20, it is preferable to acquire the time for which the cells are collected into the collection container, that is, the image data of cells within a predetermined period after the cells are collected into the collection container from a plurality of cells with high priority. According to this embodiment, the time when image data is acquired is limited to maintain the freshness of the cell. In particular, in a case in which the cell is a blood cell included in the human peripheral blood and the time from blood collection to gene analysis is long, the problem that the cell is not capable of being analyzed is likely to occur. The cell analysis system according to this embodiment can reduce the time until gene analysis.

In a case in which the number of cells to be subjected to gene analysis is predetermined, a time limit makes it possible to perform gene analysis before the number of cells reaches a necessary number of cells. That is, priority may be given to the freshness of the cell.

It is preferable that the control unit 40 determines whether to analyze the cells collected in the collection container 20 on the basis of the image data. In some cases, the cell is not the target cell from the acquisition result of the image data. In a case in which the control unit 40 determines whether to analyze the cells, it is possible to analyze only the target cell. Gene analysis can be given as an example of the cell analysis.

It is preferable to analyze at least one of the shape, color, size, and staining intensity of the cell on the basis of the image data. For example, the analysis of these elements makes it possible to perform gene analysis without an error.

Next, an analysis process for determining a good nucleated red blood cell to be subjected to gene analysis will be described.

The priority ranking of gene analysis is determined from the size of the cells on the basis of the image data. For example, in a case in which the size of the cell is very small, it can be determined that a cell is not stored in the well, but dust is stored in the well. In addition, in a case in which the size of the cell is very large, it can be determined that a plurality of cells are likely to be stored in one well. In a case in which the size of the cell is appropriate, it can be determined that the possibility of one cell being stored in the well is high.

A rank indicating the possibility that the cell will be a red blood cell is determined from the color of the cell on the basis of the image data. In a case in which the color of the cell is red, the cell is a red blood cell and the possibility that the cell will be a nucleated red blood cell is high. Therefore, the cell ranks high as a collection target. In contrast, in a case in which the color of the cell is white, the possibility that the cell will be a white blood cell or a neutrophil cell is high. Therefore, the cell ranks low as the collection target.

The priority ranking of gene analysis is determined from the shape (form) of the cell on the basis of image data. In a case in which the shape of the cell is a circle, the cell can be determined to be a good red blood cell. In a case in which the shape of the cell is not a circle, the cell can be determined to be a white blood cell or a damaged red blood cell.

The control unit 40 can determine whether the cell is suitable for gene analysis, using at least one of the above-mentioned analysis processes or a combination of the analysis processes.

In this embodiment, the case in which the cell is a blood cell has been described as an example. However, the invention is not limited thereto. In addition, the cell collected into the well has been described. However, the cell may be collected into a tube.

### Explanation of References

- 1:: cell analysis system
- 10:: detection device
- 20:: collection container
- 30:: imaging apparatus
- 40:: control unit
- 50:: personal computer
- 52:: keyboard
- 54:: display
- 102:: nozzle
- 104:: flow cell
- 106:: light source
- 108:: detector
- 110:: detector
- 112, 114:: deflection electrode plate
- 202:: well
- 204:: side wall
- 206:: identification mark
- 208:: tube
- 210:: support member
- 212:: hole
- 302:: first light source
- 304:: table
- 306:: lens
- 308:: excitation filter
- 310:: dichroic mirror
- 312:: fluorescence filter
- 314:: second light source
- 316:: imaging device

## Claims

1. A cell analysis system (1) comprising:
a detection device (10) that detects staining intensity of a stained cell;
a collection container (20) that collects a plurality of cells whose staining intensity has been detected by the detection device;
a control unit (40) that is configured to determine priority in acquiring image data on the basis of a detection result of the staining intensity of the stained cell; and
an imaging apparatus (30) that acquires the image data of the cells collected into the collection container on the basis of the priority determined by the control unit.

2. The cell analysis system according to claim 1,
wherein the control unit determines image data acquisition conditions on the basis of the detection result of the staining intensity of the stained cell.

3. The cell analysis system according to claim 2,
wherein the conditions include at least one of an exposure time in a case in which the image data is acquired, an observation magnification, a wavelength of illumination light, or a wavelength of observation light.

4. The cell analysis system according to any one of claims 1 to 3,
wherein the control unit limits a number of cells whose image data is to be acquired.

5. The cell analysis system according to claim 4,
wherein the control unit limits a number of cells whose image data is to be acquired, on the basis of the detection result of the staining intensity of the stained cell and the number of cells collected into the collection container.

6. The cell analysis system according to claim 4,
wherein the control unit limits a number of cells whose image data is to be acquired, on the basis of the detection result of the staining intensity of the stained cell and a time for which the cells are collected into the collection container.

7. The cell analysis system according to any one of claims 1 to 6,
wherein the cell is stained with a plurality of types of dyes.

8. The cell analysis system according to any one of claims 1 to 7,
wherein the control unit determines whether to analyze the cells collected into the collection container on the basis of the image data.

9. The cell analysis system according to any one of claims 1 to 8,
wherein the cell is a blood cell.

10. The cell analysis system according to any one of claims 1 to 9,
wherein at least one of the shape, color, size, or staining intensity of the cell is analyzed on the basis of the image data.

## Patentansprüche

1. Zellanalysesystem (1) aufweisend:
eine Detektionsvorrichtung (10), die die Färbungsintensität einer gefärbten Zelle detektiert;
einen Sammelbehälter (20), der eine Mehrzahl von Zellen sammelt, deren Färbungsintensität von der Detektionsvorrichtung detektiert wurde;
eine Kontrolleinheit (40), die dazu ausgelegt ist, eine Rangordnung bei der Erfassung von Bilddaten auf der Basis eines Detektionsergebnisses der Färbungsintensität der gefärbten Zelle zu bestimmen; und
eine Bildverarbeitungsapparatur (30), die die Bilddaten der in dem Sammelbehälter gesammelten Zellen auf der Basis der von der Kontrolleinheit bestimmten Rangordnung erfasst.

2. Zellanalysesystem nach Anspruch 1,
wobei die Kontrolleinheit Bilddaten-Erfassungsbedingungen auf der Basis des Detektionsergebnisses der Färbungsintensität der gefärbten Zelle bestimmt.

3. Zellanalysesystem nach Anspruch 2,
wobei die Bedingungen mindestens eine der Bedingungen Expositionszeit in einem Fall, in dem die Bilddaten erfasst werden, Betrachtungs-Vergrößerung, Wellenlänge des Beleuchtungslichts oder Wellenlänge des Betrachtungslichts umfasst.

4. Zellanalysesystem nach einem der Ansprüche 1 bis 3,
wobei die Kontrolleinheit die Anzahl an Zellen, deren Bilddaten erfasst werden sollen, begrenzt.

5. Zellanalysesystem nach Anspruch 4,
wobei die Kontrolleinheit die Anzahl an Zellen, deren Bilddaten erfasst werden sollen, auf der Basis des Detektionsergebnisses der Färbungsintensität der gefärbten Zelle und der Anzahl an in dem Sammelbehälter gesammelten Zellen begrenzt.

6. Zellanalysesystem nach Anspruch 4,
wobei die Kontrolleinheit die Anzahl an Zellen, deren Bilddaten erfasst werden sollen, auf der Basis des Detektionsergebnisses der Färbungsintensität der gefärbten Zelle und der Zeit, während der die Zellen in dem Sammelbehälter gesammelt werden, begrenzt.

7. Zellanalysesystem nach einem der Ansprüche 1 bis 6,
wobei die Zelle mit einer Mehrzahl von Arten von Farbstoffen gefärbt wird.

8. Zellanalysesystem nach einem der Ansprüche 1 bis 7,
wobei die Kontrolleinheit auf der Basis der Bilddaten bestimmt, ob die in dem Sammelbehälter gesammelten Zellen analysiert werden sollen.

9. Zellanalysesystem nach einem der Ansprüche 1 bis 8,
wobei die Zelle eine Blutzelle ist.

10. Zellanalysesystem nach einem der Ansprüche 1 bis 9,
wobei mindestens eine der Eigenschaften Form, Farbe, Größe oder Färbungsintensität der Zelle auf der Basis der Bilddaten analysiert wird.

## Revendications

1. Système d'analyse cellulaire (1), comprenant :
un dispositif de détection (10), lequel détecte une intensité de coloration d'une cellule colorée ;
un récipient de collecte (20), lequel collecte une pluralité de cellules dont l'intensité de coloration a été détectée par le dispositif de détection ;
une unité de commande (40), laquelle est configurée pour déterminer une priorité quant à l'acquisition de données d'image sur la base d'un résultat de détection de l'intensité de coloration de la cellule colorée, et
un appareil d'imagerie (30), lequel acquiert les données d'image des cellules collectées dans le récipient de collecte sur la base de la priorité déterminée par l'unité de commande.

2. Système d'analyse cellulaire selon la revendication 1,
dans lequel l'unité de commande détermine des conditions d'acquisition de données d'image sur la base du résultat de détection de l'intensité de coloration de la cellule colorée.

3. Système d'analyse cellulaire selon la revendication 2,
dans lequel les conditions incluent au moins un paramètre parmi un temps d'exposition dans un cas où les données d'image sont acquises, un grossissement d'observation, une longueur d'onde de lumière d'éclairage, ou une longueur d'onde de lumière d'observation.

4. Système d'analyse cellulaire selon l'une quelconque des revendications 1 à 3,
dans lequel l'unité de commande limite un nombre de cellules dont les données d'images doivent être acquises.

5. Système d'analyse cellulaire selon la revendication 4,
dans lequel l'unité de commande limite un nombre de cellules dont les données d'images doivent être acquises, sur la base du résultat de détection de l'intensité de coloration de la cellule colorée et du nombre de cellules collectées dans le récipient de collecte.

6. Système d'analyse cellulaire selon la revendication 4,
dans lequel l'unité de commande limite un nombre de cellules dont les données d'images doivent être acquises, sur la base du résultat de détection de l'intensité de coloration de la cellule colorée et d'un temps durant lequel les cellules sont collectées dans le récipient de collecte.

7. Système d'analyse cellulaire selon l'une quelconque des revendications 1 à 6,
dans lequel la cellule est colorée avec une pluralité de types de colorants.

8. Système d'analyse cellulaire selon l'une quelconque des revendications 1 à 7,
dans lequel l'unité de commande détermine d'analyser ou ne pas analyser les cellules collectées dans le récipient de collecte sur la base des données d'image.

9. Système d'analyse cellulaire selon l'une quelconque des revendications 1 à 8,
dans lequel la cellule est un globule sanguin.

10. Système d'analyse cellulaire selon l'une quelconque des revendications 1 à 9,
dans lequel au moins un des paramètres parmi la forme, la couleur, la taille, ou l'intensité de coloration de la cellule est analysé sur la base des données d'image.
